# EUROPÄISCHE PATENTSCHRIFT

(11) **EP 1 575 430 B1**
(45) Veröffentlichungstag und Bekanntmachung des Hinweises auf die Patenterteilung: **27.05.2009**
(21) Anmeldenummer: 03789202.3
(22) Anmeldetag: 09.12.2003
(51) Int. Cl.: A61B 10/00

(54) **OPTISCHES BIOPSIEINSTRUMENT**
OPTICAL BIOPSY INSTRUMENT
INSTRUMENT DE BIOPSIE OPTIQUE

(30) Priorität: 10.12.2002 DE 10258483
(43) Veröffentlichungstag der Anmeldung: 21.09.2005
(73) Patentinhaber: Charité - Universitätsmedizin Berlin, 10117 Berlin (DE)
(72) Erfinder: HÜNERBEIN, Michael, 10719 Berlin (DE)
(74) Vertreter: Gulde, Klaus W.
(86) Internationale Anmeldenummer: PCT/EP2003/013995
(87) Internationale Veröffentlichungsnummer: WO 2004/052210

(56) Entgegenhaltungen:
- US-A- 4 620 547
- US-A- 5 681 277
- US-A- 5 762 613
- US-A1- 2001 047 183
- US-A1- 2003 181 823
- LOVE S M ET AL: "Breast-duct endoscopy to study stages of cancerous breast disease" LANCET THE, LANCET LIMITED. LONDON, GB, Bd. 348, Nr. 9033, 12. Oktober 1996 (1996-10-12), Seiten 997-999, XP002091339 ISSN: 0140-6736 in der Anmeldung erwähnt

## Beschreibung

Die Erfindung betrifft ein optisches Biopsieinstrument, das geeignet ist zur Entnahme von Gewebeproben aus kleinen Gangsystemen, insbesondere aus Milchgängen der Brust, sowie Verfahren zur Entnahme von Gewebeproben unter Verwendung des Biopsieinstruments.

Brustkrebs stellt mit einem Anteil an der Krebssterblichkeit von etwa 20 % die häufigste krebsbedingte Todesursache bei Frauen in Deutschland und damit ein erhebliches medizinisches und sozioökonomisches Problem dar. Für eine günstige Prognose und erfolgreiche Therapie des Brustkrebses ist die Früherkennung von enormer Wichtigkeit. So konnte in internationalen Studien belegt werden, dass durch Früherkennungsprogramme mit (röntgenologischem) Mammographie-Screening eine signifikante Reduktion der Brustkrebssterblichkeit erzielt werden kann.

Jedoch wurde in den letzten Jahren eine deutliche Zunahme von intraduktalen Karzinomen, das sind Karzinome der Milchgänge (Ductus lactiferi) der Mamma, von 4 auf über 20 % festgestellt (Bässler, Onkologie 4, 1998, 878-895). Daher sind diese Tumore des Milchgangsystems gegenwärtig in den Mittelpunkt der Bemühungen zur Verbesserung der Diagnostik und Therapie des Mammakarzinoms gerückt. Die frühe Diagnose dieser kleinen Tumore ist jedoch insbesondere wegen ihrer Eigenart, sich langsam in dem präformierten Gangsystem der Brust auszubreiten, problematisch und mit konventionellen radiologischen Verfahren (Mammographie) oder Ultraschall (Mammasonographie) oft nur unzureichend möglich. Die klinischen Symptome und mammographischen Befunde führen nur bei 40 bis 60 % der Patientinnen zu einer Diagnose (Bässler, Onkologie 4, 1998, 878-895).

Die endoskopische Untersuchung der Milchgänge der Brust, die so genannte Duktoskopie, könnte die Effektivität der Früherkennung insbesondere der intraduktalen Karzinome verbessern. Sie war jedoch lange Zeit wegen des geringen Querschnitts des Ductus nicht möglich. Erst die Miniaturisierung von Endoskopen hat die direkte optische Untersuchung dieser kleinen Gangsysteme ermöglicht. Im Unterschied zu anderen Organen existieren aber bisher nur wenig Erfahrungen zur Mikroendoskopie der Milchgange, wobei diese Untersuchungen der Lokalisation intraduktaler Papillome oder anderen Zwecken dienten (z.B. Shen et al., Am. Cancer Soc. 89, 2000, 1512-1519; Okazaki et al., Eur. Radiol. 9, 1999, 583-590; Love and Barsky, Lancet 348, 1996, 997-999; Rimbach et al, Zentralbl. Gynakol. 117, 1995, 198-203; Okazaki et al., Jpn. J. Clin. Oncol. 21, 1991, 188-193). Die meisten dieser Methoden gestatten jedoch keine Gewebeprobeentnahme, sondern allenfalls eine Epithelzellgewinnung durch Lavage des Ductus. Sofern eine Biopsiemöglichkeit vorgesehen ist, erfolgt diese blind und nicht unter Sicht. Gerade die genaue, optisch kontrollierte Biopsiemöglichkeit ist aber für eine optimierte Ausbreitungsdiagnostik zur Verbesserung der Therapieplanung und Therapieergebnisse auch in Hinblick auf eine brusterhaltene Therapie bei Mammakarzinomen dringend erforderlich.

Shen et al. (Surg. Endosc. 15, 2001, 1340-1345) beschreiben eine Biopsiemethode für Milchgänge, bei der ein Fiberendoskop mit einer herkömmlichen aufgeschobenen dünnen Biopsiekanüle in den Milchgang unter Sicht eingeführt wird, das Endoskop entfernt und mittels einer eingeführten sehr dünnen Spritze Gewebe aspiriert wird. Problematisch ist aber auch hier, dass die eigentliche Biopsie nicht unter optischer Kontrolle erfolgt. Hierdurch ergibt sich die Gefahr von Fehlerquellen, da bereits kleinste Abweichungen vom angesteuerten Biopsieort zu unzuverlässigen Diagnosen führen können.

Andere Publikationen beschreiben Biopsieinstrumente für andere Zwecke, die ebenfalls über keine direkte endoskopische Kontrolle der Probeentnahme verfügen. Die US PS 4,651,753 beschreibt ein Biopsieinstrument, das zur Verwendung in Verbindung mit einem Endoskop bestimmt ist. Das Biopsieinstrument umfasst im Wesentlichen eine zylindrische äußere Hülse mit einer ovalen seitlichen Öffnung, eine koaxial in der äußeren Hülse angeordnete und axial bewegliche flexible Schneidkanüle, die an ihrem vorderen Ende ein Schneidmesser trägt, sowie einen koaxial in der Schneidkanüle angeordneten beweglichen Schubschlauch. Eine Probenentnahme erfolgt, indem das Instrument durch ein Endoskop an die relevante Biopsiestelle geführt wird, mittels eines erzeugten Unterdrucks eine zu entnehmende Gewebeprobe durch die seitliche Öffnung in das Innere des Instruments angesaugt und durch Vorschiebung der Schneidkanüle abgetrennt wird und mit dem Schubschlauch in eine vordere Kammer des äußeren Zylinders befördert wird. Nachteilig an diesem Instrument ist der komplizierte Aufbau, der einer Miniaturisierung, wie sie etwa für den Einsatz in Milchgängen erforderlich ist, entgegensteht. Zudem ist keine unmittelbare endoskopische Beobachtung der Probenentnahme möglich. Ein ähnliches und daher die gleichen Nachteile aufweisendes Prinzip wird in der US PS 5,526,822 beschrieben.

Aus der US 01/0047183 A ist ein Biopsieinstrument bekannt, das eine Doppelkanüle umfasst, wobei die beiden parallelen Kanülengänge (Arbeitskanal und Vakuumkanal) über Vakuumlöcher miteinander verbunden sind. Zur Biopsieentnahme wird durch Unterdruck Gewebe durch eine seitlich im Arbeitskanal angeordnete Öffnung angesaugt und mithilfe einer schlauchförmigen, rotierenden und axial in dem Arbeitskanal beweglichen Schneidröhre abgetrennt. Genau wie das in der US 4,651,753 beschriebene Instrument ist auch hier keine Biopsieentnahme unter endoskopischer Beobachtung möglich.

US 5,681,277 A beschreibt ein optisches Instrument, das einen Katheter umfasst, in dem eine Fiberoptik verschiebbar angeordnet ist. Bei dem Katheter handelt es sich um einen massiven Körper, in dem sich verschiedene Führungsbohrungen für die Behandlungsinstrumente, zur Ableitung von Spülflüssigkeit sowie für die Fiberoptik befinden. Der Katheter weist an seinem distalen Ende eine seitliche Vertiefung auf, in dessen Bereich die Führungsbohrung für die Fiberoptik endet und die der Eröffnung eines Sichtfelds dient. Die Probenentnahme erfolgt durch einen Arbeitskanal, der am distalen Ende des Katheders endet, mithilfe eines in diesem geführten Greifinstruments.

Aus US 4,620,547 A ist ein optisches Biopsieinstrument benannt, das eine im Wesentlichen zylindrische Kanüle mit einer seitlichen, zur Aufnahme und Abtrennung einer Gewebeprobe ausgestalteten Öffnung aufweist, und ein innerhalb der Kanüle axial bewegliches Endoskop mit deutlich geringerem Außendurchmesser gegenüber einem Innendurchmesser der Kanüle. Die Abtrennung einer Gewebeprobe erfolgt durch eine Schneidkante eines axial innerhalb der Kanüle beweglichen Schneidrohres. Das Instrument ist für Probenentnahmen des Uterus ausgelegt und somit sehr engen Gangsystemen nicht zugänglich.

Zusammenfassend lässt sich feststellen, dass der klinische Nutzen der vielversprechenden Technik der Duktoskopie durch die bislang geringe optische Qualität, den fehlenden Arbeitskanal derzeit verfügbaren Instrumente und insbesondere die nicht vorhandene Möglichkeit der sichtkontrollierten Probenentnahme stark eingeschränkt wird. Hierdurch wird die klinische Akzeptanz und die breite Anwendung der Methode derzeit verhindert.

Der vorliegenden Erfindung liegt daher die Aufgabe zugrunde, ein Biopsieinstrument mit einfachem Aufbau zur Verfügung zu stellen, das Biopsieentnahmen unter unmittelbarer optischer Überwachung auch in engsten Hohlräumen, insbesondere in Milchgängen der Mamma erlaubt.

Erfindungsgemäß wird diese Aufgabe durch ein im Wesentlichen zweiteilig aufgebautes, optisches Biopsieinstrument mit den Merkmalen des Anspruchs 1 gelöst. Das erfindungsgemäße Biopsieinstrument umfasst
(a) eine im Wesentlichen zylindrische Kanüle mit einem proximalen Ende und einem distalen Ende, wobei die Kanüle mindestens eine seitliche, zur Aufnahme und Abtrennung einer Gewebeprobe ausgestaltete Öffnung aufweist, und
(b) ein innerhalb der Kanüle axial bewegliches Endoskop, wobei
ein äußerer Durchmesser des Endoskops im Wesentlichen einem inneren Durchmesser der Kanüle entspricht derart, dass durch eine axiale Relativbewegung allein des Endoskops und der Kanüle eine Abtrennung einer in die Öffnung einbringbaren Gewebeprobe ermöglicht ist.

Im Rahmen der vorliegenden Erfindung wird unter einer "im Wesentlichen zylindrischen Kanüle" ein Hohlkörper verstanden, der durch einen Mantel mit im Wesentlichen kreisrundem Querschnitt gebildet wird. Ferner bezeichnet der Begriff "seitliche Öffnung" eine beliebig ausgestaltete Lochöffnung mit geschlossenem Umfang.

Das Instrument zeichnet sich somit durch einen sehr einfachen Aufbau aus, der erstmals eine unmittelbare, endoskopisch kontrollierte Probenentnahme durch die seitliche Öffnung der Kanüle auch in sehr feinen Gefäßen oder Gangsystemen, insbesondere in den Milchgängen der Brust gestattet. Häufige Fehlerquellen bei den bislang blind durchgeführten Biopsien werden somit vermieden. Mit lediglich zwei wesentlichen Elementen, Kanüle und Endoskop, gelingt sowohl die Endoskopie als auch die Biopsie, da das mit der Öffnung zusammenwirkende Endoskop die Durchführung aller für die Probenentnahme notwendigen Handlungen, insbesondere dem Schneiden, gestattet. Die einfache Bauweise ermöglicht zudem eine sehr kleine Dimensionierung des Geräts und damit die Zugänglichkeit engster Gangsysteme.

Eine Abtrennung der zu entnehmenden Gewebeprobe von dem übrigen Gewebe wird vorzugsweise dadurch vereinfacht, dass die mindestens eine seitliche Öffnung der Kanüle an ihrem dem distalen Ende und/oder an ihrem dem proximalen Ende zugewandten Umfang zumindest bereichsweise einen Schneidbereich aufweist. Auf diese Weise kann ein einfaches Abtrennen der Gewebeprobe durch geeignete Bewegung der Kanüle und des Endoskops relativ zueinander oder nur der Kanüle erfolgen. Diese Vorgehensweisen werden noch im Einzelnen erläutert.

Der Schneidbereich der seitlichen Öffnung kann vorteilhaft durch einen Anschliff des Umfangs der Öffnung und oder durch eine Zahnung des Umfangs realisiert sein. Denkbar ist auch eine Kombination aus Anschliff und Zahnung. Die Öffnung selbst kann beispielsweise eine im Wesentlichen runde, ovale, elliptische oder rechteckige Gestaltung aufweisen.

Erfindungsgemäß entspricht ein äußerer Durchmesser des Endoskops im Wesentlichen einem inneren Durchmesser der Kanüle, das heißt ein Spiel zwischen diesen Komponenten ist möglichst gering. Eine solche relative Dimensionierung der zusammenwirkenden Komponenten zueinander erleichtert einerseits das Schneiden und anderseits die Erzeugung eines Unterdrucks im Kanülenraum, was zusätzlich dadurch erleichtert wird, dass die Kanüle an ihren distalen Ende mit einer Wandung verschlossen ist, die vorzugsweise sichtdurchlässig ist.

Das Endoskop kann wahlweise ein starres oder ein flexibles Endoskop sein, das heißt sowohl Spiegel- als auch Glasfaserendoskope finden Anwendung. Jedoch hat sich das starre Endoskop wegen seiner leichteren Handhabung beim Schneiden als besonders vorteilhaft erwiesen.

Verschiedene Vorgehensweisen zur Entnahme Gewebeproben von Gangsystemen unter Verwendung des erfindungsgemäßen Biopsieinstruments sind denkbar.

Nach einer ersten Variante wird
(a) ein optisches Biopsieinstrument, umfassend
   - eine im Wesentlichen zylindrische Kanüle mit einem proximalen Ende und einem distalen Ende, wobei die Kanüle mindestens eine seitliche, zur Aufnahme und Abtrennung einer Gewebeprobe ausgestaltete Öffnung aufweist, und
   - ein innerhalb der Kanüle axial bewegliches Endoskop, wobei ein äußerer Durchmesser des Endoskops und ein innerer Durchmesser der Kanüle derart zueinander dimensioniert sind dass ein Zusammenwirken des Endoskops mit der mindestens einen seitlichen Öffnung der Kanüle zur Abtrennung einer in die seitliche Öffnung einbringbare Gewebeprobe ermöglicht wird,
   unter endoskopischer Sicht bis zu einer Biopsiestelle in den Gang eingeführt,
(b) die Gewebeprobe durch die offen liegende Öffnung in ein Inneres der Kanüle gebracht und
(c) die Gewebeprobe von dem übrigen Gewebe abgetrennt, indem das Endoskop über die seitliche Öffnung hinweg vorgeschoben wird und/oder die Kanüle zurückgezogen wird, bis die seitliche Öffnung geschlossen ist.

Nach dieser ersten Variante erfolgt die Abtrennung der Gewebeprobe somit durch ein Gegeneinanderverschieben von Kanüle und Endoskop, so dass die Gewebeprobe durch einen durch das Endoskop ausgeübten Drucks gegen den Schneidbereich der Öffnung abgetrennt wird.

Nach einer zweiten Variante wird die Kanüle bei zurückgezogenem Endoskop, das heißt bei freiliegender Öffnung, gleichsam als Hobel verwendet, um die Abtrennung der Gewebeprobe zu bewirken. Dazu wird
(a) das oben beschriebene optische Biopsieinstrument unter endoskopischer Sicht in den Gang eingeführt, bis die seitliche Öffnung über der Biopsiestelle zu liegen kommt, und
(b) die Gewebeprobe durch die offen liegende Öffnung in ein Inneres der Kanüle gebracht und
(c) die Gewebeprobe von dem übrigen Gewebe abgetrennt, indem die Kanüle mitsamt dem festliegenden Endoskop bei freiliegender seitlicher Öffnung unter Ausübung leichten manuellen Drucks gegen die Gewebeprobe vorbewegt oder zurückbewegt wird.

Weitere bevorzugte Ausgestaltungen der Erfindung ergeben sich aus den übrigen, in den Unteransprüchen genannten Merkmalen.

Die Erfindung wird nachfolgend in einem Ausführungsbeispiel anhand der zugehörigen Zeichnungen näher erläutert. Es zeigen: -
- Figur 1: ein mikroendoskopisches System, bestehend -aus einem Endoskop, einem Videohandle und einer Lichtquelle,
- Figur 2: eine perspektivische Seitenansicht einer erfindungsgemäßen Kanüle,
- Figur 3a bis 3f: Seitenansichten erfindungsgemäßer Kanülen mit unterschiedlichen Gestaltungen der Öffnung und ihrer Schneidbereiche,
- Figur 4a bis 4d: Schritte eines erfindungsgemäßen Verfahrens zur Biopsie einer Gewebeprobe nach einer ersten Variante, und
- Figur 5a bis 5d: Schritte eines erfindungsgemäßen Verfahrens zur Biopsie einer Gewebeprobe nach einer zweiten Variante.

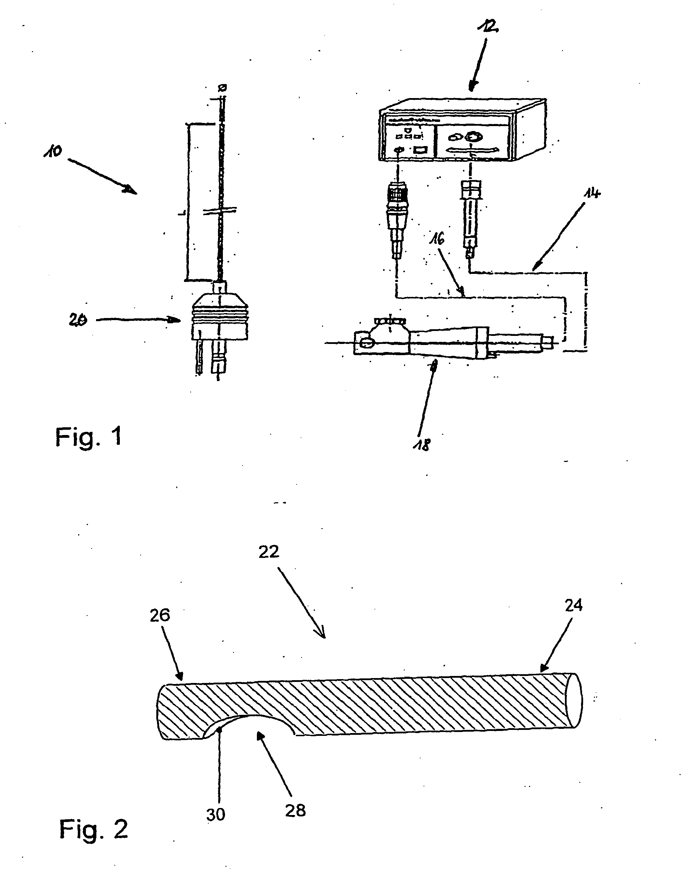

Figur 1 stellt einen Überblick über ein mikroendoskopisches System dar, wie es in der vorliegenden Erfindung zum Einsatz kommt. Das System umfasst ein Endoskop 10 (Duktoskop), das aufgrund seiner kleinen Dimensionierung für die Endoskopie enger Kanäle, insbesondere der Milchgänge der Brust (Ductus lactiferi), geeignet ist. Es handelt sich dabei um ein starres Endoskop (der Firma Volpi AG, Schweiz) mit einem äußeren Durchmesser ∅ von 1 mm und einer Länge L von 6 cm. Das Endoskop basiert auf der so genannten Gradient-Index-Technik (GRIN) und bietet eine deutlich höhere optische! Auflösung und bessere Helligkeitsausbeute gegenüber fiberoptischen Endoskopen.

Das System umfasst ferner eine Lichtquelle 12, beispielsweise eine Metallhalogenidlichtquelle. An die Lichtquelle 12 anschließbar sind ein Lichtleiterkabel 14 sowie ein Kamerakabel 16, die zu einem Videohandle 18 führen. Das Videohandle 18 weist einen hier nicht im Detail dargestellten Kamerakopf auf, der eine CCD-Kamera mit einer Auflösung von 625 Linien und einem manuellen Fokus (2 mm - ∞) beinhaltet. Die CCD-Kamera liefert ein digitales Bildsignal hervorragender Qualität. Über einen Anschlusskopf 20 ist das Endoskop 10 mit dem Videohandle 18 anschließbar und somit über das Lichtleiterkabel 14 und dem Kamerakabel 16 mit der Lichtquelle 12 verbindbar.

Das System ist in Hinblick auf seine guten optischen Eigenschaften sowie seiner einfachen Manövrierbarkeit für den breiten klinischen Einsatz geeignet. Obwohl das Endoskop über einen Arbeitskanal verfügt, durch den beispielsweise eine Lavage durchführbar ist, gestattet der geringe Durchmesser keine Biopsie. Dieses Problem wird erfindungsgemäß durch Verwendung einer auf das Endoskop 10 aufsteckbaren Kanüle 22 gelöst, die in Figur 2 dargestellt ist.

Die Kanüle 22, die im Wesentlichen die Gestalt eines Hohlzylinders hat, verfügt über ein proximales Ende 24 sowie ein distales Ende 26. Die Kanüle kann beispielsweise aus einem rigiden Kunststoff oder aus Metall sein. Ihr innerer Durchmesser ist vorzugsweise geringfügiger größer gewählt als der Außendurchmesser des Endoskops 10, so dass nur wenig Spiel zwischen Endoskop 10 und aufgeschobener Kanüle 22 vorhanden ist. Im konkreten Fall wurde eine Kanüle 22 mit einem Außendurchmesser von 1,2 mm verwendet. Die Kanüle 22 weist nahe der Kanülenspitze, das heißt im Bereich ihres distalen Endes 26, eine seitliche Öffnung 28 auf, die zumindest bereichsweise mit einem Schneidbereich 30 ausgestattet ist.

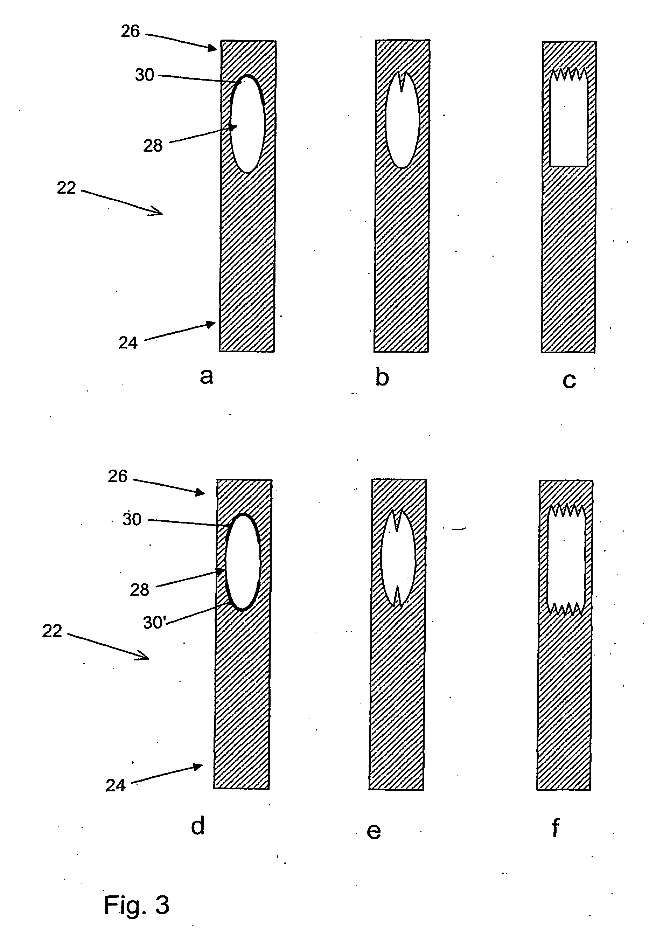

Die Figuren 3a bis 3f zeigen mehrere Ausführungsbeispiele der Kanüle 22, die sich hinsichtlich der Gestaltung der seitlichen Öffnung 28 sowie der Art des Schneidbereichs 30 unterscheiden. Die Öffnungen 28 gemäß der Figuren a, b, d und e besitzen eine im Wesentlichen ovale Gestaltung, wohingegen die Öffnungen 28 gemäß der Figuren c und f weitestgehend rechteckig geformt sind. Andere hier nicht dargestellte Gestaltungen der Öffnung 28, beispielsweise mit runder oder elliptischer Kontur, sind ebenfalls denkbar. Ebenfalls können mehr als eine seitliche Öffnung 28, beispielsweise zwei einander gegenüberliegende Öffnungen 28 vorhanden sein.

Die drei in der Figur oben abgebildeten Varianten (3a bis 3c) weisen Schneidbereiche 30 lediglich auf der dem distalen Ende 26 der Kanüle 22 zugewandten Bereich der Öffnung 28 auf. Die unteren drei Varianten (3d bis 3f) besitzen dagegen zusätzlich auch auf der dem proximalen Ende 24 der Kanüle 22 zugewandten Bereich der Öffnung 28 Schneidbereiche 30'. Ebenfalls denkbar ist ein über den gesamten Umfang der Öffnung 28 umtaufender Schneidbereich oder für bestimmte Handhabungen des Biopsieinstrumentes lediglich Schneidbereiche 30' auf der proximalen Seite.

Bei den Varianten 3a und 3d sind die Schneidbereiche 30, 30' in Form eines Anschliffs der Kanüle 22 realisiert (angedeutet durch fette Linien). Auf der anderen Seite bestehen die Schneidbereiche 30, 30' der übrigen Varianten in einer Zahnung, wobei die Schneidbereiche 30, 30' der Varianten 3b und 3e in Form jeweils eines einzelnen Zahns gestaltet sind und die der Varianten 3c und 3f in Form einer feineren Mehrfachzahnung. Es kann daneben auch vorteilhaft sein, Anschliff und Zahnung miteinander zu kombinieren, etwa durch einen gezahnten Anschliff oder durch eine Zahnung auf der einen und einen Anschliff auf der anderen Seite der Öffnung.

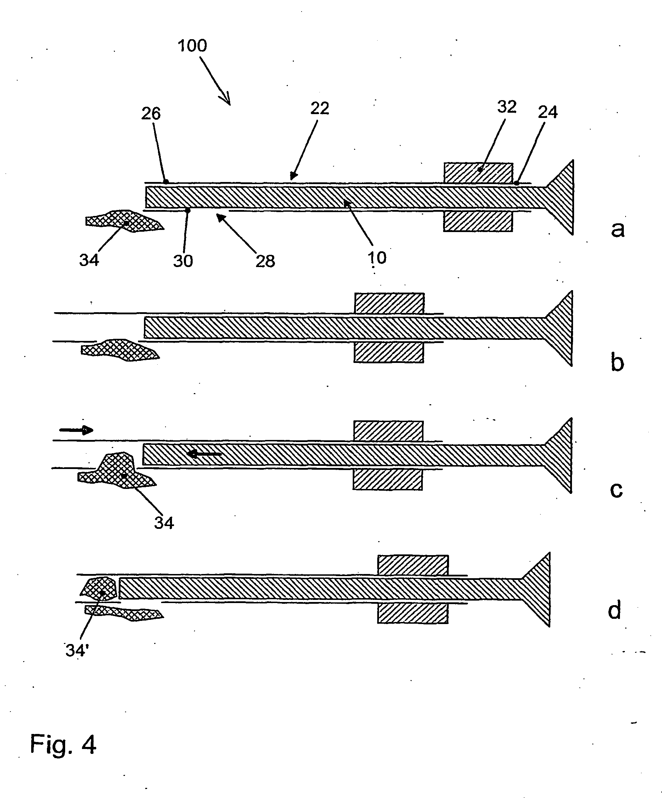

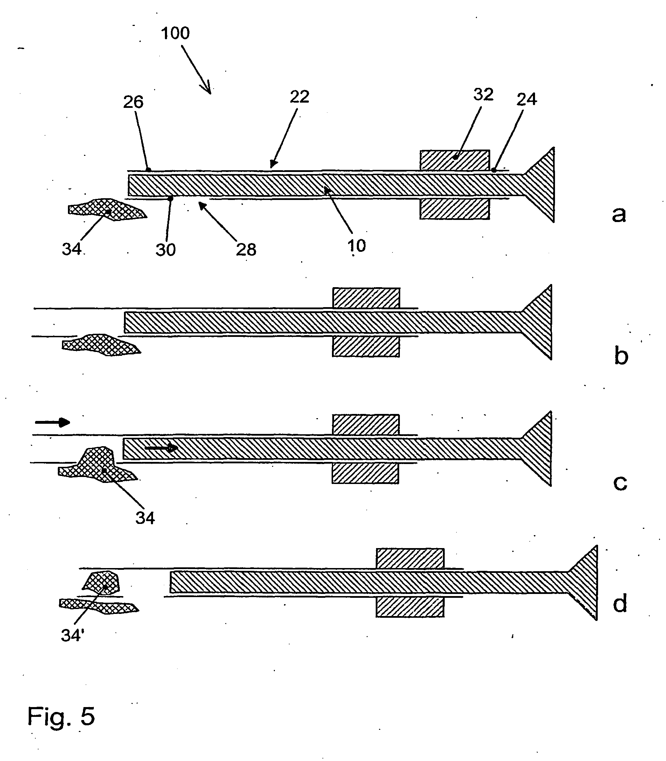

Zwei verschiedene Methoden zur Biopsie einer Gewebeprobe unter Verwendung des erfindungsgemäßen Biopsieinstruments sind schematisch in den Figuren 4 und 5 dargestellt.

Gemäß der ersten, in Figur 4 skizzierten Methode wird das insgesamt mit 100 bezeichnete Biopsieinstrument, bestehend aus dem Endoskop 10 und der auf diesem aufgeschobenen Kanüle 22 unter endoskopischer Sichtkontrolle in ein zu untersuchendes Hohlgefäß (nicht dargestellt), insbesondere in einen Milchgang eingeführt (Figur 4a). Vorzugsweise befindet sich in dieser Phase das Endoskop 10 in einer maximal vorgeschobenen Position innerhalb der Kanüle 22, so dass die distale Kanülenspitze im Wesentlichen mit der Spitze des Endoskops 10 abschließt. Zur besseren Handhabung verfügt die Kanüle 22 an ihrem proximalen Bereich 24 über einen Griff 32. Sobald eine zu entnehmende Gewebeprobe 34 in den Sichtbereich des Endoskops 10 gelangt, wird die Einführbewegung des Instruments 100 beendet.

Anschließend wird mit einem Handgriff die Kanüle 22 so weit vorgeschoben, bis die Öffnung 28 der Kanüle 22 frei wird, das heißt nicht länger durch das innen liegende Endoskop 10 verschlossen ist, und die Öffnung 28 über der Gewebeprobe 34 zu liegen kommt (Figur 4b).

Im nächsten Schritt wird die zu entnehmende Gewebeprobe 34 durch die Öffnung 28 in das Innere der Kanüle 22 gebracht (Figur 4c). Dies kann einerseits spontan infolge einer gegen die Kanüle 22 und der Öffnung 28 wirkenden Gewebespannung geschehen. Optional kann das Eindringen des Gewebes durch einen in der Kanüle 22 erzeugten leichten Unterdruck unterstützt werden, wobei der Unterdruck durch das zuvor erfolgte Vorschieben der Kanüle 22 erzeugt werden kann. Für diesen Fall kann die distale Kanülenspitze mit einer nicht dargestellten sichtdurchlässigen Wandung verschlossen sein kann. Optional kann der Unterdruck auch durch den vorhandenen Arbeitskanal des Endoskops 10 erzeugt werden.

Sobald die Gewebeprobe 34 in die Kanüle eingedrungen ist, erfolgt ein Abtrennen von dem restlichen Gewebe, indem das Endoskop 10 vorgeschoben oder die Kanüle 22 zurückgezogen wird. Es ist auch vorteilhaft möglich, beide Bewegungen miteinander zu kombinieren, indem Kanüle 22 und Endoskop 10 gegeneinander bewegt werden (siehe Pfeile in Figur 4c). Aufgrund der scherenden Einwirkung des Schneidbereichs 30 der Öffnung 28 der Kanüle 22 kommt es dabei zu einem Einklemmen und einer Abtrennung des Gewebes, wobei die abgetrennte Gewebeprobe 34' in das Innere des distalen Bereichs 26 der Kanüle 22 geschoben wird (Figur 4d). Sodann kann das gesamte Biopsieinstrument 100 mitsamt der Gewebeprobe 34' aus dem Gangsystem entfernt werden. Auch hierfür kann der Verschluss der Kanülenspitze durch ein Fenster vorteilhaft sein, um einen Verlust der Gewebeprobe 34' beim Herausziehen des Instruments 100 zu verhindern.

Eine zweite Variante der Methode zur Biopsie einer Gewebeprobe ist in Figur 5 dargestellt. Einführen des Biopsieinstruments 100 (Figur 5a) und Einbringen der Gewebeprobe 34 (Figuren 5b und 5c) erfolgen analog der zuvor in Figur 4 beschriebenen Methode. Der Unterschied hierzu ist in der Art des Abtrennens der Gewebeprobe 34 zu sehen, das durch eine hobelartige Verwendung der Kanüle 22 erfolgt. Hierzu wird, sobald die Gewebeprobe 34 in die Kanüle 22 eingedrungen ist, das gesamte Instrument 100, bestehend aus Kanüle 22 und hierin festgelegtem Endoskop 10 in einer gemeinsamen Richtung (siehe Pfeile in Figur 5c) unter leichter manuellen Druckausübung gegen die Gewebeprobe 34 bewegt, so dass der Schneidbereich 30 der Kanüle 22 an die Gewebeprobe 34 angreift und diese abtrennt (Figur 5d). Je nachdem, ob sich der Schneidbereich 30 an der distalen oder der proximalen Seite der Öffnung 28 befindet, kann die gleichgerichtete Abtrennbewegung in die eine oder die andere Richtung erfolgen. In dem dargestellten Beispiel, wo der Schneidbereich 30 an der distalen Seite der Öffnung 28 angeordnet ist, erfolgt die Gewebeäbtrennung durch Zurückziehen des Instruments 100.

Für eine hier nicht dargestellte weitere Vorgehensweise der Biopsieentnahme ist eine zweite seitliche Öffnung der Kanüle 22 vorteilhaft. Hierbei wird bei zurückgezogenem Endoskop 10 die Kanüle 22 mit einer Hebelbewegung derart auf die zu entnehmende Gewebeprobe 34 gedrückt, dass das Gewebe durch die erste seitliche Öffnung 28 in das Kanüleninnere eindringt und durch die gegenüberliegende zweite Öffnung teilweise wieder aus der Kanüle 22 in den Ductus austritt. Das Abtrennen der Probe 34 erfolgt dann entweder wieder durch ein Gegeneinanderbewegen von Endoskop 10 und Kanüle 22 gemäß Figur 4 oder durch einen, hebelartigen Einsatz des Instruments 100 gemäß Figur 5. Ein Schneidbereich 30 an der zweiten seitlichen Öffnung ist für diese Variante nicht unbedingt erforderlich.

Der gesamte Prozess der Probenentnahme nach allen beschriebenen Methoden kann unter optimaler endoskopischer Sicht überwacht und gesteuert werden. Insbesondere kann mittels des erfindungsgemäßen Biopsieinstruments 100 der Entnahmeort sehr gezielt angesteuert werden. Fehlproben, die bislang bei Blindbiopsie durch geringfügiges Verfehlen des Entnahmeortes häufig waren, werden nunmehr vermieden.

Aufgrund der hohen medizinischen und sozioökonomischen Relevanz von Brustkrebs ist davon auszugehen, dass das erfindungsgemäße Biopsieinstrument sowie die Verfahren für seine Anwendung bei Ärzten und Patientinnen große Akzeptanz finden werden. Die Methode ist einfach und preiswert und kann nach entsprechender kurzer Ausbildung der Ärzte flächendeckend eingesetzt werden. Da es sich bei dem Biopsieinstrument um ein Produkt im Niedrigpreis handelt, kann es sowohl in Kliniken als auch im niedergelassenen Bereich eingesetzt werden.

### BEZUGSZEICHEN

- 10: Endoskop
- 12: Lichtquelle
- 14: Lichtleiterkabel
- 16: Kamerakabel
- 18: Videohandle mit Kamerakopf
- 20: Anschlusskopf
- 22: Kanüle
- 24: proximales Ende
- 26: distales Ende
- 28: Öffnung
- 30: Schneidbereich
- 32: Griff
- 34: Gewebeprobe

## Patentansprüche

1. Optisches Biopsieinstrument (100), umfassend
(a) eine im Wesentlichen zylindrische Kanüle (22) mit einem proximalen Ende (24) und einem distalen Ende (26), wobei die Kanüle (22) mindestens eine seitliche Öffnung (28) aufweist, die zur Aufnahme und Abtrennung einer Gewebeprobe ausgestaltet ist, und
(b) ein innerhalb der Kanüle (22) axial bewegliches Endoskop (10),
**dadurch gekennzeichnet, dass**
ein äußerer Durchmesser des Endoskops (10) im Wesentlichen einem inneren Durchmesser der Kanüle (22) entspricht derart, dass durch eine axiale Relativbewegung allein des Endoskops (10) und der Kanüle (22) eine Abtrennung einer in die Öffnung (28) einbringbaren Gewebeprobe (34) ermöglicht ist.

2. Optisches Biopsieinstrument (100) nach Anspruch 1, **dadurch gekennzeichnet, dass** die mindestens eine seitliche Öffnung (28) der Kanüle (22) an ihrem dem distalen Ende (26) zugewandten Bereich und/oder an ihrem dem proximalen Ende (24) zugewandten Bereich zumindest bereichsweise einen Schneidbereich (30) aufweist.

3. Optisches Biopsieinstrument (100) nach Anspruch 2, **dadurch gekennzeichnet, dass** der Schneidbereich (30) durch einen Anschliff des Umfangs der mindestens einen seitlichen Öffnung (28) oder durch eine Zahnung des Umfangs realisiert ist oder durch beide Maßnahmen gleichzeitig.

4. Optisches Biopsieinstrument (100) nach einem der vorhergehenden Ansprüche, **dadurch gekennzeichnet, dass** die mindestens eine seitliche Öffnung (28) eine im Wesentlichen runde, ovale, elliptische oder rechteckige Gestaltung aufweist.

5. Optisches Biopsieinstrument (100) nach einem der vorhergehenden Ansprüche, **dadurch gekennzeichnet, dass** die Kanüle (22) an ihrem distalen Ende (26) mit einer Wandung, insbesondere mit einer transparenten Wandung, verschlossen ist.

6. Optisches Biopsieinstrument (100) nach einem der vorhergehenden Ansprüche, **dadurch gekennzeichnet, dass** der äußere Durchmesser des Endoskops (10) und der innere Durchmesser der Kanüle (22) relativ zueinander derart dimensioniert sind, das infolge eines Vorschiebens der Kanüle (22) relativ zu dem Endoskop (10) ein Unterdruck im Kanülenraum erzeugbar ist.

7. Optisches Biopsieinstrument (100) nach einem der vorhergehenden Ansprüche, **dadurch gekennzeichnet, dass** ein Außendurchmesser der Kanüle (22) maximal 1,2 mm beträgt.

8. Optisches Biopsieiristrument (100) nach einem der vorhergehenden Ansprüche, **dadurch gekennzeichnet, dass** das Endoskop ein starres Endoskop oder ein flexibles Glasfaserendoskop ist.

## Claims

1. Optical biopsy instrument (100), comprising
(a) a substantially cylindrical cannula (22) with a proximal end (24) and a distal end (26), the cannula (22) having at least one lateral opening (28) configured for receiving and separating a tissue sample, and
(b) an endoscope (10) which is axially movable inside the cannula (22),
**characterised in that** an outer diameter of the endoscope (10) substantially corresponds to an inner diameter of the cannula (22) such that as the result of an axial relative movement alone of the endoscope (10) and of the cannula (22) it is possible to separate a tissue sample (34) which can be introduced into the opening (28).

2. Optical biopsy instrument (100) according to Claim 1, **characterised in that** the at least one lateral opening (28) of the cannula (22) has a cutting region (30) at least in certain parts of its region facing the distal end (26) and/or of its region facing the proximal end (24).

3. Optical biopsy instrument (100) according to Claim 2, **characterised in that** the cutting region (30) is produced by a ground edge of the circumference of the at least one lateral opening (28) or by a toothing of the circumference or by both measures simultaneously.

4. Optical biopsy instrument (100) according to any one of the preceding claims, **characterised in that** the at least one lateral opening (28) has a substantially round, oval, elliptic or rectangular shape.

5. Optical biopsy instrument (100) according to any one of the preceding claims, **characterised in that** the cannula (22) is sealed at its distal end (26) by a wall, in particular by a transparent wall.

6. Optical biopsy instrument (100) according to any one of the preceding claims, **characterised in that** the outer diameter of the endoscope (10) and the inner diameter of the cannula (22) are configured relative to one another such that as the result of the cannula (22) being advanced relative to the endoscope (10), it is possible to produce a reduced pressure in the cannula chamber.

7. Optical biopsy instrument (100) according to any one of the preceding claims, **characterised in that** an outer diameter of the cannula (22) is at the most 1.2 mm.

8. Optical biopsy instrument (100) according to any one of the preceding claims, **characterised in that** the endoscope is a rigid endoscope or a flexible glass fibre endoscope.

## Revendications

1. Instrument de biopsie optique (100), comprenant
(a) une canule (22) essentiellement cylindrique comportant une extrémité proximale (24) et une extrémité distale (26), la canule (22) présentant au moins une ouverture latérale (28) qui est conçue pour le prélèvement et la séparation d'un échantillon de tissu, et
(b) un endoscope (10) pouvant se déplacer axialement dans la canule (22),
**caractérisé en ce que**
un diamètre externe de l'endoscope (10) correspond essentiellement à un diamètre interne de la canule (22) de telle sorte que par un mouvement axial relatif unique de l'endoscope (10) et de la canule (22), une séparation d'un échantillon de tissu pouvant être inséré dans l'ouverture (28) soit possible.

2. Instrument de biopsie optique (100) selon la revendication 1, **caractérisé en ce qu'**au moins une ouverture latérale (28) de la canule (22) présente au niveau de la zone tournée vers l'extrémité distale (26) et/ou au niveau de la zone tournée vers l'extrémité proximale (24), une zone coupante (30) au moins par endroits.

3. Instrument de biopsie optique (100) selon la revendication 2, **caractérisé en ce que** la zone coupante (30) est réalisée par le poli du pourtour d'au moins l'ouverture latérale (28) ou par une denture du pourtour ou par ces deux mesures concomitantes.

4. Instrument de biopsie optique (100) selon l'une des revendications précédentes, **caractérisé en ce qu'**au moins l'ouverture latérale (28) présente une configuration essentiellement arrondie, ovale, elliptique ou rectangulaire.

5. Instrument de biopsie optique (100) selon l'une des revendications précédentes, **caractérisé en ce que** la canule (22) est fermée au niveau de son extrémité distale (26) par une paroi, en particulier par une paroi transparente.

6. Instrument de biopsie optique (100) selon l'une des revendications précédentes, **caractérisé en ce que** le diamètre externe de l'endoscope (10) et le diamètre interne de la canule (22) sont dimensionnés l'un par rapport à l'autre de telle sorte qu'en conséquence d'un avancement de la canule (22) par rapport à l'endoscope (10), une dépression puisse être produite dans l'espace de canule.

7. Instrument de biopsie optique (100) selon l'une des revendications précédentes, **caractérisé en ce qu'**un diamètre externe de la canule (22) mesure au maximum 1,2 mm.

8. Instrument de biopsie optique (100) selon l'une des revendications précédentes, **caractérisé en ce que** l'endoscope est un endoscope rigide ou un endoscope souple à fibres de verre.
